# FASCICULE DE BREVET EUROPEEN

(11) **EP 1 879 856 B1**
(45) Date de publication et mention de la délivrance du brevet: **16.03.2011**
(21) Numéro de dépôt: 06755462.6
(22) Date de dépôt: 28.04.2006
(51) Int. Cl.: C07C 271/04, C07C 271/12, A61P 25/00, A61P 31/00, A61P 35/00, A61K 31/27

(54) **DERIVES D'ALKYL-, ALKENYL- ET ALKYNYLCARBAMATES, LEUR PREPARATION ET LEUR APPLICATION EN THERAPEUTIQUE**
ALKYL-, ALKENYL- UND ALKYNYLCARBAMAT-DERIVATE, DEREN HERSTELLUNG UND THERAPEUTISCHE VERWENDUNG
ALKYL-, ALKENYL- AND ALKYNYL CARBAMATE DERIVATIVES, THE PREPARATION AND THERAPEUTIC USE THEREOF

(30) Priorité: 03.05.2005 FR 0504492
(43) Date de publication de la demande: 23.01.2008
(73) Titulaire: Sanofi-Aventis, 75013 Paris (FR)
(72) Inventeur: ALMARIO-GARCIA, Antonio, F-92290 Chatenay-malabry (FR); GEORGE, Pascal, c/o sanofi-aventis, 75013 Paris (FR); HOORNAERT, Christian, c/o sanofi-aventis, F- 75013 Paris (FR); LI, Adrien-Tak, F-92260 Fontenay Aux Roses (FR); PUECH, Frédéric, c/o sanofi-aventis, 75013 Paris (FR)
(74) Mandataire: Le Coupanec, Pascale A.M.P.
(86) Numéro de dépôt international: PCT/FR2006/000958
(87) Numéro de publication internationale: WO 2006/117461

(56) Documents cités:
- FR-A- 2 843 964
- FR-A- 2 854 633
- FR-A- 2 860 514
- TARZIA G ET AL: "Design, synthesis, and structure-activity relationships of alkylcarbamic acid aryl esters, a new class of Fatty Acid Amide Hydrolase inhibitors" JOURNAL OF MEDICINAL AND PHARMACEUTICAL CHEMISTRY, AMERICAN CHEMICAL SOCIETY. EASTON, US, vol. 46, no. 12, 2003, pages 2352-2360, XP002257137

## Description

L'invention se rapporte à des dérivés d'alkyl-, d'alkènyl- et d'alkynylcarbamates, à leur préparation et leur application en thérapeutique.

Les composés de l'invention répondent à la formule générale (I) 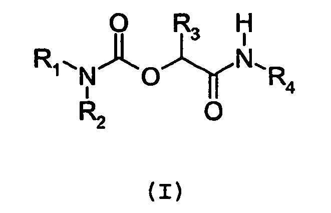 dans laquelle
R₁ représente un groupe C₅₋₂₆-alkyle, C₅₋₂₆-alkènyle, C₅₋₂₆-alkynyle ou un groupe carboné aliphatique polyinsaturé comprenant entre 5 et 26 atomes de carbone et comprenant au moins deux insaturations choisies parmi les doubles et les triples liaisons ;
R₂ représente un atome d'hydrogène ou un groupe C₁₋₆-alkyle ;
R₃ représente un atome d'hydrogène ou un groupe C₁₋₃-alkyle ;
R₄ représente un atome d'hydrogène ou un groupe C₁₋₄-alkyle, C₃₋₇-cycloalkyle, C₃₋₇-cycloalkyl-C₁₋₃-alkyle les groupes alkyle, alkènyle, alkynyle et carboné aliphatique polyinsaturé pouvant être linéaires, ramifiés ou cycliques.

Les composés de formule générale (I) peuvent comporter un ou plusieurs carbones asymétriques. Ils peuvent exister sous forme d'énantiomères ou de diastéréoisomères. Les composés de formule générale (I) peuvent également exister sous forme de stéréoisomères *cis* ou *trans.* Ces énantiomères, diastéréoisomères et stéréoisomères, ainsi que leurs mélanges, y compris les mélanges racémiques, font partie de l'invention.

Les composés de formule générale (I) peuvent exister sous forme d'hydrates ou de solvats, à savoir sous forme d'associations ou de combinaisons avec une ou plusieurs molécules d'eau ou avec un solvant. De tels hydrates et solvats font également partie de l'invention.

Dans le cadre de la présente invention, on entend par :
- C_{t-z} où t et z peuvent prendre les valeurs de 1 à 26, une chaîne carbonée pouvant avoir de t à z atomes de carbone, par exemple C₁₋₃ une chaîne carbonée pouvant avoir de 1 à 3 atomes de carbone ;
- un groupe alkyle : un groupe aliphatique saturé, linéaire, ramifié ou cyclique ; par exemple un groupe C₁₋₃-alkyle représente une chaîne carbonée qui peut avoir de 1 à 3 atomes de carbone, par exemple un méthyle, éthyle, propyle, isopropyle, cyclopropyle, etc ;
- un groupe alkènyle : un groupe alkyle, linéaire, ramifié ou cyclique, comprenant une double liaison, par exemple un groupe C₅₋₂₆-alkènyle représente une chaîne carbonée de 5 à 26 atomes de carbone, linéaire, ramifiée ou cyclique, comprenant une double liaison, par exemple un pentènyle, cyclopentènyle, hexènyle, cyclohexènyle, heptènyle, cycloheptènyle, octènyle, cyclooctènyle, etc ;
- un groupe alkynyle : un groupe alkyle linéaire, ramifié ou cyclique, comprenant une triple liaison, par exemple un groupe C₅₋₂₆-alkynyle représente une chaîne carbonée de 5 à 26 atomes de carbone linéaire, ramifié ou cyclique, comprenant une triple liaison, par exemple un hexynyle, heptynyle, octynyle, etc ;
- un groupe carboné aliphatique polyinsaturé : un groupe alkyle linéaire, ramifié ou cyclique, comprenant au moins deux insaturations choisies parmi les doubles et les triples liaisons, par exemple un pentadiènyle, hexadiènyle, cyclohexadiènyle, heptadiènyle, heptatriènyle, cycloheptadiènyle, cycloheptatriènyle, un pentadiynyle, hexadiynyle, heptadiynyle.

Parmi les composés de formule générale (I) objets de l'invention, un premier groupe de composés est constitué par les composés pour lesquels :
- R₁ représente
   un groupe C₅₋₂₆-alkyle, plus particulièrement un pentyle, hexyle, heptyle, octyle, cyclooctyle, 1-méthylheptyle, nonyle, décyle, 1-méthyldécyle, dodécyle, cyclododécyle, tétradécyle, 1-méthyltridécyle, pentadécyle, octadécyle, nonadécyle,
   un groupe C₅₋₂₆-alkènyle, plus particulièrement un hexènyle, octènyle, tétradécènyle, hexadécènyle, octadécènyle, icosènyle,
   ou un groupe carboné aliphatique polyinsaturé comprenant entre 5 et 26 atomes de carbone et comprenant au moins deux insaturations choisies parmi les doubles liaisons, plus particulièrement un octadécatriènyle, nonadécatétraènyle, icosadiènyle, icosatriènyle, icosatétraènyle ;
- et/ou R₂ représente un atome d'hydrogène ou un groupe C₁₋₆-alkyle, plus particulièrement un méthyle ou un hexyle ;
- et/ou R₃ représente un atome d'hydrogène ou un groupe C₁₋₃-alkyle ;
- et/ou R₄ représente un atome d'hydrogène ou un groupe C₁₋₄-alkyle, C₃₋₇-cycloalkyle, C₃₋₇-cycloalkyl-C₁₋₃-alkyle.

Parmi les composés de ce premier groupe, on peut citer les composés de formule (I) pour lesquels :
R₃ représente un atome d'hydrogène ou un groupe C₁₋₃-alkyle, plus particulièrement un méthyle ;
et/ou R₄ représente un atome d'hydrogène ou un groupe C₁₋₄-alkyle, plus particulièrement un méthyle.

Parmi les composés de ce premier groupe, on peut notamment citer les composés suivants :
- Pent-1-yl carbamate de 2-amino-1-méthyl-2-oxoéthyle
- (2E)-Hex-2-èn-1-yl carbamate de 2-(méthylamino)-2-oxoéthyle
- (2E)-Hex-2-èn-1-yl carbamate de 2-amino-2-oxoéthyle
- (2Z)-Hex-2-èn-1-yl carbamate de 2-amino-2-oxoéthyle
- (3Z)-Hex-3-èn-1-yl carbamate de 2-amino-2-oxoéthyle
- (3E)-Hex-3-èn-1-yl carbamate de 2-amino-2-oxoéthyle
- (3Z)-Hex-3-èn-1-yl carbamate de 2-(méthylamino)-2-oxoéthyle
- (3E)-Hex-3-èn-1-yl carbamate de 2-(méthylamino)-2-oxoéthyle
- (2Z)-Hex-2-èn-1-yl carbamate de 2-(méthylamino)-2-oxoéthyle
- Hex-1-yl carbamate de 2-(méthylamino)-2-oxoéthyle
- Hex-1-yl carbamate de 2-amino-1-méthyl-2-oxoéthyle
- Dihex-1-yl carbamate de 2-(méthylamino)-2-oxoéthyle
- Hept-1-yl carbamate de 2-(méthylamino)-2-oxoéthyle
- (2E)-Oct-2-èn-1-yl carbamate de 2-(méthylamino)-2-oxoéthyle
- (2E)-Oct-2-èn-1-yl carbamate de 2-amino-2-oxoéthyle
- (3Z)-Oct-3-èn-1-yl carbamate de 2-(méthylamino)-2-oxoéthyle
- (3Z)-Oct-3-èn-1-yl carbamate de 2-amino-2-oxoéthyle
- (SZ)-Oct-5-èn-1-yl carbamate de 2-amino-2-oxoéthyle
- (5Z)-Oct-5-èn-1-yl carbamate de 2-(méthylamino)-2-oxoéthyle
- Oct-7-èn-1-yl carbamate de 2-amino-2-oxoéthyle
- Oct-7-èn-1-yl carbamate de 2-(méthylamino)-2-oxoéthyle
- Cyclooct-1-yl carbamate de 2-(méthylamino)-2-oxoéthyle
- Oct-1-yl carbamate de 2-amino-1-méthyl-2-axoéthyle
- 1-Mêthylhept-1-yl carbamate de 2-(méthylamino)-2-oxoéthyle
- Non-1-yl carbamate de 2-(méthylamino)-2-oxoéthyle
- Déc-1-yl carbamate de 2-(méthylamino)-2-oxoéthyle
- Déc-1-yl carbamate de 2-amino-1-méthyl-2-oxoéthyle
- 1-Méthyldéc-1-yl carbamate de 2-(mêthylamino)-2-oxoéthyle
- Cyclododéc-1-yl carbamate de 2-(méthylamino)-2-oxoéthyle
- Dodéc-1-yl carbamate de 2-(méthylamino)-2-oxoéthyle
- (11Z)-Tetradéc-11-èn-1-yl carbamate de 2-amino-2-oxoéthyle
- (11Z)-Tetradéc-11-èn-1-yl carbamate de 2-(méthylamino)-2-oxoéthyle
- Tétradéc-1-yl carbamate de 2-(méthylamino)-2-oxoéthyle
- (9Z)-Hexadêc-9-èn-1-yl carbamate de 2-amino-2-oxoéthyle
- (9Z)-Hexadéc-9-èn-1-yl carbamate de 2-(méthylamino)-2-oxoéthyle
- 1-Méthyltridécyl carbamate de 2-(méthylamino)-2-oxoéthyle
- Pentadéc-1-yl carbamate de 2-(méthylamino)-2-oxoéthyle
- (6E,9E,12E)-Octadéca-6,9,12-trièn-1-yl carbamate de 2-(méthylamino)-2-oxoéthyle
- (6Z)-octadéc-6-èn-1-yl carbamate de 2-amino-2-oxoéthyle
- (6Z)-Octadéc-6-èn-1-yl carbamate de 2-(méthylamino)-2-oxoéthyle
- (méthyl)octadéc-1-yl carbamate de 2-(méthylamino)-2-oxoéthyle
- (4Z,7Z,10Z,13Z)-Nonadéca- 4,7,10,13-têtraèn-1-yl carbamate de 2-amino-2-oxoéthyle
- Nonadéc-1-yl carbamate de 2-(méthylamino)-2-oxoéthyle
- (52,82,11Z,14Z) -Icosa-5,8,11,14-tétraèn-1-yl carbamate de 2-(méthylamino)-2-oxoéthyle
- (11E,14E,17E)-Icosa-11,14,17-trièn-1-yl carbamate de 2-(méthylamino)-2-oxoéthyle
- (11E,14E)-Icosa-11,14-dièn-1-yl carbamate de 2-(méthylamino)-2-oxoéthyle
- (11E)-Icosa-11-èn-1-yl carbamate de 2-(méthylamino)-2-oxoéthyle.

Parmi les composés de formule générale (I) objets de l'invention, un second groupe de composés est constitué par les composés pour lesquels :
- R₁ représente un groupe C₁₀₋₂₀-alkyle, C₁₀₋₂₀-alkènyle, C₁₀₋₂₀-alkynyle ou un groupe carboné aliphatique polyinsaturé comprenant entre 10 et 20 atomes de carbone et comprenant au moins deux insaturations choisies parmi les doubles et les triples liaisons ;
- et/ou R₂ représente un atome d'hydrogène ou un groupe C₁₋₆-alkyle, plus particulièrement un méthyle ou un hexyle ;
- et/ou R₃ représente un atome d'hydrogène ou un groupe C₁₋₃-alkyle ;
- et/ou R₄ représente un atome d'hydrogène ou un groupe C₁₋₄-alkyle, C₃₋₇-cycloalkyle, C₃₋₇-cycloalkyl-C₁₋₃-alkyle.

Parmi les composés de ce second groupe, on peut citer les composés de formule (I) pour lesquels :
- R₁ représente un groupe C₁₀₋₂₀-alkyle, plus particulièrement un décyle, méthyldécyle, dodécyle, dyclododécyle, méthyltridécyle, tétradécyle, pentadécyle, octadécyle, nonadécyle, un groupe C₁₀₋₂₀-alkènyle, plus particulièrement un tétradécènyle, hexadécènyle, octadécènyle, icosaènyle, un groupe carboné aliphatique polyinsaturé comprenant entre 10 et 20 atomes de carbone et comprenant au moins deux insaturations choisies parmi les doubles liaisons, plus particulièrement un octadécatriènyle, nonadécatétraènyle, icosadiènyle, icosatriènyle, icosatétraènyle ;
- et/ou R₂ représente un atome d'hydrogène ou un groupe C₁₋₆-alkyle, plus particulièrement un méthyle ;
- et/ou R₃ représente un atome d'hydrogêne ou un groupe C₁₋₃-alkyle, plus particulièrement un méthyle ;
- et/ou R₄ représente un atome d'hydrogène ou un groupe C₁₋₄-alkyle, plus particulièrement un méthyle.

Parmi les composés du sous-groupe défini ci-dessus, les composés suivants peuvent être cités :
- Déc-1-yl carbamate de 2-(méthylamino)-2-oxoéthyle
- 1-Méthyldéc-1-yl carbamate de 2-(méthylamino)-2-oxoéthyle
- Cyclododéc-1-yl carbamate de 2-(méthylamino)-2-oxoéthyle
- Dodéc-1-yl carbamate de 2-(méthylamino)-2-oxoéthyle
- (11Z)-Tetradêc-11-èn-1-yl carbamate de 2-amino-2-oxoéthyle
- (11Z)-Tetradéc-11-èn-1-yl carbamate de 2-(méthylamino)-2-oxoêthyle
- Tétradéc-1-yl carbamate de 2-(méthylamino)-2-oxoéthyle
- (9Z)-Hexadéc-9-èn-1-yl carbamate de 2-amino-2-oxoéthyle
- (9Z)-Hexadéc-9-èn-1-yl carbamate de 2-(méthylamino)-2-oxoéthyle
- 1-Méthyltridécyl carbamate de 2-(méthylamino)-2-oxoéthyle
- Pentadéc-1-yl carbamate de 2-(méthylamino)-2-oxoéthyle
- (6E,9E,12E)-Octadéca-6,9,12-trièn-1-yl carbamate de 2-(méthylamino)-2-oxoéthyle
- (6Z)-Octadéc-6-èn-1-yl carbamate de 2-(méthylamino)-2-oxoéthyle
- (5Z,8Z,11Z,14Z)-Icosa-5,8,11,14-tétraèn-1-yl carbamate de 2-(méthylamino)-2-oxoéthyle
- (11E,14E,17E)-Icosa-11,14,17-trièn-1-yl carbamate de 2-(méthylamino)-2-oxoéthyle
- (11E,14E)-Icosa-11,14-dièn-1-yl carbamate de 2-(méthylamino)-2-oxoéthyle.

Les composés de l'invention peuvent être préparés selon différentes méthodes, illustrées par les schémas qui suivent.

Ainsi selon une première méthode (schéma 1), les composés de formule générale (I) peuvent être préparés en faisant réagir une amine de formule générale (II), dans laquelle R₁ et R₂ sont tels que définis dans la formule générale (I), avec un carbonate de formule générale **(III)** dans laquelle Z représente un atome d'hydrogène ou un groupe nitro tandis que R₃ et R₄ sont tels que définis dans la formule générale (I).

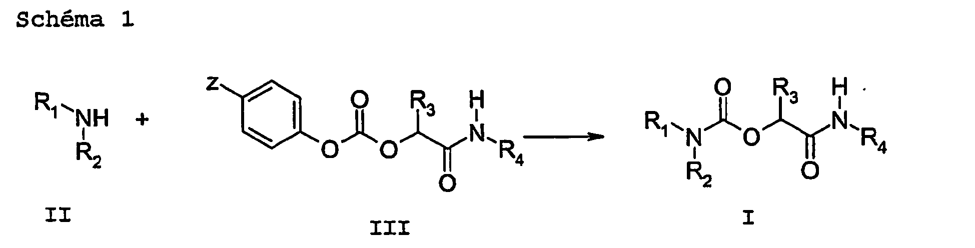

Les carbonates de formule générale (III) peuvent être préparés selon toute méthode décrite dans la littérature, par exemple par réaction d'un alcool de formule générale HOCHR₃CONR₄ où R₃ et R₄ sont tel que défini dans la formule générale (I), avec le chloroformiate de phényle ou de 4-nitrophényle, en présence d'une base telle que la triéthylamine ou la diisopropyléthylamine.

Une autre méthode (Schéma 2) d'obtention des composés de formule générale (I) pour lesquels R₂ représente un atome d'hydrogène, consiste à faire réagir un composé de formule générale (IV) dans laquelle R₁ est tel que défini dans la formule générale (I) et W représente un groupement hydroxy, mésylate, tosylate, ou un atome de chlore, de brome ou d'iode, avec une oxazolidine-dione de structure générale (V) dans laquelle R₃ est tel que défini dans la formule générale (I) pour fournir le dérivé oxazolidine-dione de structure générale (VI).

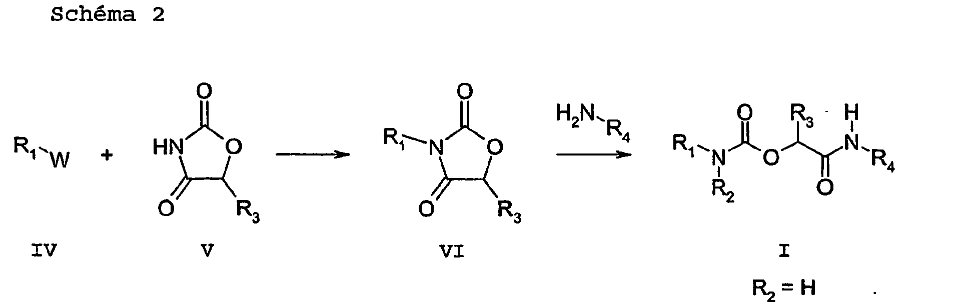

Dans le cas où W représente un groupement hydroxy, la réaction peut être conduite suivant les conditions de Mitsunobu (Synthesis 1981, 1-28), par exemple, par action de l'azodicarboxylate de diéthyle ou diisopropyle en présence de triphénylphosphine. Dans le cas où W représente un atome de chlore, de brome, ou d'iode, ou un groupement mésylate ou tosylate, la réaction peut être conduite en présence d'une base telle que la 1,1,3,3-tétraméthylguanidine, l'hydrure de sodium ou le tert-butoxyde de sodium dans un solvant tel que le tétrahydrofurane, l'acétonitrile ou le diméthylformamide à une température comprise entre 0°C et 80°C. Le dérivé oxazolidine-dione de formule générale (VI) ainsi obtenu est ensuite transformé en composé de formule générale (I) pour lequel R₂ représente un atome d'hydrogène, par aminolyse au moyen d'une amine de formule générale R₄NH₂ où R₄ est tel que défini dans la formule générale (I).

Les composés de formule générale (II),(IV) et (V) ainsi que les alcools de formule générale HOCHR₃CONR₄ et les amines de formule générale R₄NH₂, quand leur mode de préparation n'est pas décrit, sont disponibles dans le commerce ou décrits dans la littérature,ou peuvent être préparés selon différentes méthodes décrites dans la littérature ou connues de l'homme du métier.

L'invention, selon un autre aspect de ses aspects, a également pour objet les composés de formules (VI). Ces composés sont utiles comme intermédiaires de synthèse des composés de formule (I).

Les exemples qui suivent illustrent la préparation de quelques composés de l'invention. Ces exemples ne sont pas limitatifs et ne font qu'illustrer l'invention. Les microanalyses, les spectres I.R et R.M.N et/ou la LC-MS (liquid chromatography coupled to Mass Spectroscopy) confirment les structures et les puretés des composés obtenus.

PF(°C) représente le point de fusion en degrés Celsius. Les numéros indiqués entre parenthèses dans les titres des exemples correspondent à ceux de la 1^{ère} colonne du tableau ci-après.

### Exemple 1 : Décyl carbamate de 2-(méthylamino)-2-oxoéthyle (composé n°26)

Une solution de 136 mg (0,65 mmole) de 2-(méthylamino)-2-oxoéthyl carbonate de phényle et de 133 mg (0,85 mmole) de décylamine dans 2 mL de dichlorométhane est agitée à température ambiante pendant 18 h.

Le solvant est évaporé sous pression réduite et le résidu d'évaporation est chromatographié sur colonne de gel de silice en éluant avec un mélange de 2% de méthanol dans le dichlorométhane.

Le produit obtenu est cristallisé dans l'éther diisopropylique pour conduire à 135 mg de solide blanc.
LC-MS : M+H = 273
PF(°C) : 98-100°C
RMN¹H (CDCl₃) δ (ppm) : 6,10 (signal large, 1H) ; 4,80 (signal large, 1H) ; 4,60 (s, 2H) ; 3,20 (q, 2H) ; 2,85 (d, 3H) ; 1.65-1,45 (m, 4H) ; 1,45-1,20 (m, 12H) ; 1,00-0,80 (m, 3H).

### Exemple 2 : (11Z)-tétradécyl-11-ên-1-yl carbamate de 2-(méthylamino)-2-oxoéthyle (composé n°32)

### 2.1 3-[(11Z)-tétradécyl-11-èn-1-yl]-1,3-oxazolidine-2,4-dione

A une solution de 200 mg (1,98 mmole) de 1,3-oxazolidine-2,4-dione et de 571 mg (2,18 mmoles) de triphénylphosphine dans 4 mL de tétrahydrofurane, on ajoute 420 mg (1,98 mmole) de (11*Z*)-tétradécyl-11-én-1-ol en solution dans 2 mL de tétrahydrofurane. On additionne ensuite goutte à goutte 0,99 mL (2,2 mmoles) d'une solution à 40% d'azodicarboxylate de diéthyle dans le toluène. Le mélange est agité à température ambiante pendant 5h.

Le solvant est évaporé sous pression réduite et le résidu d'évaporation est purifié par chromatographie sur colonne de gel de silice en éluant avec un gradient de 10 à 50% d'acétate d'éthyle dans le cyclohexane.

On obtient ainsi 490 mg d'une huile jaune.

RMN¹H (CDCl₃) δ (ppm) : 5,25 (m, 2H) ; 4,60 (s, 2H) ; 3,45 (t, 2H) ; 2,10-1,80 (m, 4H) ; 1,65-1,45 (m, 2H) ; 1,40-1,05 (m, 14H) ; 0,90 (t, 3H).

### 2.2 (11Z)-tétradécyl-11-èn-1-ylcarbamate de 2-(méthylamino)-2-oxoéthyle

A une solution de 240 mg (0,81 mmole) de 3-[(11*Z*)-tétradécyl-11-èn-1-yl]-1,3-oxazolidine-2,4-dione, obtenue à l'étape 2.1, dans 1,5 mL de méthanol on ajoute 6 mL d'une solution 2 M de méthylamine dans le méthanol.

Le mélange est agité pendant 2 h à température ambiante puis le solvant est évaporé sous pression réduite et le résidu d'évaporation est purifié par chromatographie sur colonne de gel de silice en éluant avec un gradient de 70 à 100% d'acétate d'étyle dans le cyclohexane.

On isole un solide jaune qui est soniqué dans un mélange de 0,5 mL d'acétate d'éthyle et de 4 mL de cyclohexane pendant 30 minutes. Le solide est séparé par centrifugation et élimination du surnageant.

On obtient ainsi 74 mg d'un solide jaune pâle après séchage.
LC-MS : M+H = 327
PF(°C) : 79-81°C
RMN¹H (DMSO-d₆) δ (ppm) : 7,75 (s large, 1H) ; 7,15 (t large, 1H) ; 5,30 (m, 2H) ; 4,30 (s, 2H) ; 2,95 (m, 2H) ; 1,60 (d, 3H) ; 1,95 (m, 4H) ; 1,40-1,15 (m, 16H) ; 0,90 (t, 3H).

### Exemple 3 : (11Z)-tétradécyl-11-èn-1-yl carbamate de 2-amino-2-oxoéthyle (composé n°31)

A une solution de 240 mg (0,81 mmole) de 3-[(11Z)-têtradécyl-11-èn-1-yl]-1,3-oxazolidine-2,4-dione, obtenue à l'étape 2.1 de l'exemple 2, dans 1,5 mL de méthanol on ajoute 6 mL d'une solution 2 M. d'ammoniac dans le méthanol.

Le mélange est agité pendant 18 h à température ambiante puis le solvant est évaporé sous pression réduite. Le résidu d'évaporation est chromatographié sur colonne de gel de silice en éluant avec un gradient de 80 à 100% d'acétate d'éthyle dans le cyclohexane.

On isole un solide jaune qui est soniqué dans un mélange de 1 mL d'acétate d'éthyle et de 4 mL de cyclohexane pendant 30 minutes. Le solide est séparé par centrifugation et élimination du surnageant.

On obtient ainsi 99 mg d'un solide jaune pâle après séchage.
LC-MS : M+Na = 335
PF(°C) : 120-123°C
RMN¹H (DMSO-d₆) δ (ppm) : 7,30-7,05 (m, 3H) ; 5,30 (m, 2H) ; 4,30 (s, 2H) ; 2,95 (m, 2H) ; 2,00 (m, 4H) ; 1,45-1,15 (m, 16H) ; 0,90 (t, 3H).

Le tableau 1 qui suit illustre les structures chimiques et les propriétés physiques de quelques composés selon l'invention.

Dans ce tableau :
- la colonne "PF°C" renseigne les points de fusion des produits en degrés Celsius. "N.D" signifie que le point de fusion est non déterminé.
- la colonne "LC-MS ou (MS)" renseigne le résultat d'analyse des produits par LC-MS (liquid chromatography coupled to Mass Spectroscopy) réalisée sur un appareil Agilent LC-MSD Trap en mode ESI positif ou par MS (Mass Spectroscopy) sur un appareil Autospec M (EBE) en utilisant la technique DCI-NH3.

**Tableau 1**

| | | | | | | |
|---|---|---|---|---|---|---|
| 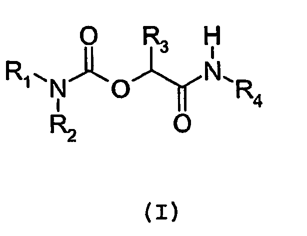 | | | | | | |

| N° | R₁ | R₂ | R₃ | R₄ | PF °C | LC-MS ou (MS) |
|---|---|---|---|---|---|---|
| 1 | Pent-1-yl | H | CH₃ | H | 132-134 | (M+H 203) |
| 2 | (2E)-Hex-2-èn-1-yl | H | H | CH₃ | 85-87 | M+Na 237 |
| 3 | (2E)-Hex-2-èn-1-yl | H | H | H | 126-128 | M+Na 223 |
| 4 | (2Z)-Hex-2-èn-1-yl | H | H | H | 102-104 | M+Na 223 |
| 5 | (3Z)-Hex-3-èn-1-yl | H | H | H | N.D | M+Na 223 |
| 6 | (3E)-Hex-3-èn-1-yl | H | H | H | 138-140 | M+Na 223 |
| 7 | (3Z)-Hex-3-èn-1-yl | H | H | CH₃ | 72-74 | M+Na 237 |
| 8 | (3E)-Hex-3-èn-1-yl | H | H | CH₃ | 88-90 | M+Na 237 |
| 9 | (2Z)-Hex-2-èn-1-yl | H | H | CH₃ | 74-76 | M+Na 237 |
| 10 | Hex-1-yl | H | H | CH₃ | 86-88 | M+H 217 |
| 11 | Hex-1-yl | H | CH₃ | H | 123-125 | (M+H 217) |
| 12 | Hex-1-yl | Hex-1-yl | H | CH₃ | N.D | M+H 301 |
| 13 | Hept-1-yl | H | H | CH₃ | 87-89 | M+H 231 |
| 14 | (2E)-Oct-2-èn-1-yl | H | H | CH₃ | 80-82 | M+Na 265 |
| 15 | (2E)-Oct-2-èn-1-yl | H | H | H | 126-128 | M+Na 251 |
| 16 | (3Z)-Oct-3-èn-1-yl | H | H | CH₃ | 62-64 | M+Na 265 |
| 17 | (3Z)-Oct-3-èn-1-yl | H | H | H | 113-115 | M+Na 251 |
| 18 | (5Z)-Oct-5-èn-1-yl | H | H | H | 122-124 | M+Na 251 |
| 19 | (5Z)-Oct-5-èn-1-yl | H | H | CH₃ | 69-71 | M+Na 265 |
| 20 | Oct-7-èn-1-yl | H | H | H | 122-124 | M+Na 251 |
| 21 | Oct-7-èn-1-yl | H | H | CH₃ | 72-74 | M+Na 265 |
| 22 | cyclooct-1-yl | H | H | CH₃ | 81-83 | M+H 243 |
| 23 | Oct-1-yl | H | CH₃ | H | 130-132 | (M+H 245) |
| 24 | 1-Méthylhept-1-yl | H | H | CH₃ | N.D | M+H 267 |
| 25 | Non-1-yl | H | H | CH₃ | 95-97 | M+H 259 |
| 26 | Déc-1-yl | H | H | CH₃ | 98-100 | M+H 273 |
| 27 | Déc-1-yl | H | CH₃ | H | 133-135 | (M+H 273) |
| 28 | 1-Méthyldéc-1-yl | H | H | CH₃ | 71-73 | M+H 287 |
| 29 | cyclododéc-1-yl | H | H | CH₃ | 171-173 | M+H 299 |
| 30 | Dodéc-1-yl | H | H | CH₃ | 103-105 | M+H.301 |
| 31 | (11Z)-Tetradéc-11-èn-1-yl | H | H | H | 120-123 | M+Na 335 |
| 32 | (11Z)-Tetradéc-11-èn-1-yl | H | H | CH₃ | 79-81 | M+H 327 |
| 33 | Tétradéc-1-yl | H | H | CH₃ | 106-108 | M+H 329 |
| 34 | (9Z)-Hexadéc-9-èn-1-yl | H | H | H | 127-129 | M+H 341 M+Na 363 |
| 35 | (9Z)-Hexadéc-9-èn-1-yl | H | H | CH₃ | 69-71 | M+H 355 M+Na 377 |
| 36 | 1-Méthyltridécyl | H | H | CH₃ | 82-84 | M+H 329 |
| 37 | Pentadéc-1-yl | H | H | CH₃ | 107-109 | M+H 343 |
| 38 | (6E,9E,12E)-Octadéca-6,9,12-trièn-1-yl | H | H | CH₃ | N.D | M+H 379 |
| 39 | (6Z)-Octadéc-6-èn-1-yl | H | H | H | 100-102 | M+H 369 M+Na 391 |
| 40 | (6Z)-Octadéc-6-èn-1-yl | H | H | CH₃ | 72-74 | M+H 383 |
| 41 | Octadéc-1-yl | CH₃ | H | CH₃ | 85-87 | M+H 399 |
| 42 | (4Z,7Z,10Z,13Z)-Nonadéca-4,7,10,13-tétraèn-1-yl | H | H | H | N.D | M+H 377 |
| 43 | Nonadéc-1-yl | H | H | H | 113-115 | M+H 399 |
| 44 | (5Z,8Z,11Z,14Z)- Icosa-5,8,11,14- tétraèn-1-yl | H | H | CH₃ | N.D | M+H 405 |
| 45 | (11E,14E,17E)-Icosa -11,14,17-trièn-1-yl | H | H | CH₃ | 57-59 | M+H 407 M+Na 429 |
| 46 | (11E,14E)-Icosa-11,14-dièn-1-yl | H | H | CH₃ | 58-60 | M+H 409 M+Na 431 |
| 47 | (11E)-Icosa-11-èn-1-yl | H | H | CH₃ | 77-79 | M+H 411 M+Na 433 |

Les composés de l'invention ont fait l'objet d'essais pharmacologiques permettant de déterminer leur effet inhibiteur de l'enzyme FAAH (Fatty Acid Amido Hydrolase).

L'activité inhibitrice a été mise en évidence dans un test radioenzymatique basé sur la mesure du produit d'hydrolyse (éthanolamine [1-³H]) de l'anandamide [éthanolamine 1-³H] par la FAAH (Life Sciences (1995), 56, 1999-2005 et Journal of Pharmacology and Experimental Therapeutics (1997), 283, 729-734*).* Ainsi, les cerveaux de souris (moins le cervelet) sont prélevés et conservés à -80°C. Les homogénats membranaires sont préparés extemporanément par homogénéisation des tissus au Polytron dans un tampon Tris-HCl 10 mM (pH 8) contenant 150 mM NaCl et 1 mM EDTA. La réaction enzymatique est ensuite conduite dans 70 µL de tampon contenant de l'albumine de sérum bovin sans acides gras (1 mg/mL). Sont ajoutés successivement les composés testés à différentes concentrations, l'anandamide [éthanolamine 1-³H] (activité spécifique de 15-20 Ci/mmol) diluée à 10 µM avec de l'anandamide froide et la préparation membranaire (400 µg tissu congelé par essai). Après 15 minutes à 25°C, la réaction enzymatique est arrêtée par addition de 140 µL de chloroforme/méthanol (2 :1). Le mélange est agité 10 minutes puis centrifugé pendant 15 minutes à 3500 g. Un aliquot (30 µL) de la phase aqueuse contenant l'éthanolamine [1-³H] est compté par scintillation liquide.

Dans ces conditions, les composés les plus actifs de l'invention présentent des CI₅₀ (concentration inhibant de 50 % l'activité enzymatique contrôle de la FAAH) comprises entre 0,001 et 1 µM.

Par exemple, les composés n° 13, 22, 24 et 47 du tableau présentent respectivement des CI₅₀ de respectivement 0,091, 0,696, 0,089 et 0,154 µM.

Il apparaît donc que les composés selon l'invention ont une activité inhibitrice sur l'enzyme FAAH.

L'activité *in vivo* des composés de l'invention a été évaluée dans un test d'analgésie.

Ainsi, l'administration intrapéritonéale (i.p.) de PBQ (phénylbenzoquinone, 2 mg/kg dans une solution de chlorure de sodium à 0,9 % contenant 5 % d'éthanol) chez des souris mâles OF1 de 25 à 30 g, provoque des étirements abdominaux, en moyenne 30 torsions ou contractions pendant la période de 5 à 15 minutes après injection. Les composés testés sont administrés par voie orale (p.o.) ou par voie intrapéritonéale (i.p.) en suspension dans du Tween 80 à 0,5 %, 60 minutes ou 120 minutes avant l'administration de PBQ. Dans ces conditions, les composés les plus puissants de l'invention réduisent de 35 à 70 % le nombre d'étirements induits par le PBQ, dans une gamme de doses comprise entre 1 et 30 mg/kg.

L'enzyme FAAH *(*Chemistry and Physics of Lipids, (2000), 108, 107-121) catalyse l'hydrolyse des dérivés endogènes d'amides et d'esters de différents acides gras tels que la N-arachidonoyléthanolamine (anandamide), la *N*-palmitoyl-éthanolamine, la N-oléoyléthanolamine, l'oléamide ou le 2-arachidonoylglycérol. Ces dérivés exercent différentes activités pharmacologiques en interagissant, entre autres, avec les récepteurs cannabinoïdes et vanilloides.

Les composés de l'invention, bloquent cette voie de dégradation et augmentent le taux tissulaire de ces substances endogènes. Ils peuvent être utilisés à ce titre dans la prévention et le traitement des pathologies dans lesquelles les cannabinoïdes endogènes et/ou tous autres substrats métabolisés par l'enzyme FAAH, sont impliqués. On peut par exemple citer les maladies et les affections suivantes :
la douleur notamment les douleurs aiguës ou chroniques de type neurogène : migraine, douleurs neuropathiques incluant les formes associées au virus de l'herpès et au diabète, les douleurs aiguës ou chroniques associées aux maladies inflammatoires : arthrite, arthrite rhumatoïde, ostéoarthrite, spondylite, goutte, vascularite, maladie de Crohn, syndrome du colon irritable, les douleurs aiguës ou chroniques périphériques, les vertiges, les vomissements, les nausées en particulier celles consécutives à une chimiothérapie, les troubles du comportement alimentaire en particulier les anorexies et cachexies de diverses natures, les pathologies neurologiques et psychiatriques : tremblements, dyskinésies, dystonies, spasticité, comportements compulsifs et obsessionnels, syndrome de Tourette, toutes les formes de dépression et d'anxiété de toute nature et origine, troubles de l'humeur, psychoses, les maladies neuro-dégénératives aiguës et chroniques : maladie de Parkinson, maladie d'Alzheimer, démence sénile, chorée de Huntington, lésions liées à l'ischémie cérébrale et aux traumatismes crâniens et médullaires, l'épilepsie, les troubles du sommeil incluant les apnées du sommeil, les maladies cardiovasculaires en particulier hypertension, arythmies cardiaques, artériosclérose, crise cardiaque, ischémies cardiaques, l'ischémie rénale, les cancers : tumeurs bénignes de la peau, papillomes et tumeurs cérébrales, tumeurs de la prostate, tumeurs cérébrales (glioblastomes, médullo-épithéliomes, médulloblastomes, neuroblastomes, tumeurs d'origine embryonnaire, astrocytomes, astroblastomes, épendyomes, oligodendrogliomes, tumeur du plexus, neuroépithéliomes, tumeur de l'épiphyse, épendymoblastomes, méningiomes malins, sarcomatoses, mélanomes malins, schwénnomes), les désordres du système immunitaire, notamment les maladies auto-immunes : psoriasis, lupus érythémateux, maladies du tissu conjonctif ou connectivites, syndrome de Sjögrer's, spondylarthrite ankylosante, spondylarthrite indifférenciée, maladie de Behcet's, anémies auto-immunes hémolytiques, sclérose en plaques, sclérose latérale amyotrophique, amyloses, rejet de greffes, maladies affectant la lignée plasmocytaire, les maladies allergiques : l'hypersensibilité immédiate ou retardée, rhinites ou conjonctivites allergiques, dermatites de contact, les maladies infectieuses parasitaires, virales ou bactériennes : SIDA, méningites, les maladies inflammatoires, notamment les maladies articulaires : arthrite, arthrite rhumatoïde, ostéoarthrite, spondylite, goutte, vascularite, maladie de Crohn, syndrome du colon irritable, l'ostéoporose, les affections oculaires : hypertension oculaire, glaucome, les affections pulmonaires : maladies des voies respiratoires, bronchospasmes, toux, asthme, bronchite chronique, obstruction chronique des voies respiratoires, emphysème, les maladies gastro-intestinales: syndrome du colon irritable, désordres inflammatoires intestinaux, ulcères, diarrhées, l'incontinence urinaire et l'inflammation vésicale.

Les composés de l'invention peuvent donc être utilisés pour la préparation de médicaments, en particulier de médicaments inhibiteurs de l'enzyme FAAH.

Ainsi, selon un autre de ses aspects, l'invention a pour objet des médicaments qui comprennent un composé de formule (I), ou un hydrate ou un solvat du composé de formule (I).

Ces médicaments trouvent leur emploi en thérapeutique, notamment dans le traitement et la prévention des pathologies ci-dessus mentionnées.

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, ou un hydrate ou un solvat dudit composé, ainsi qu'au moins un excipient pharmaceutiquement acceptable.

Lesdits excipients sont choisis selon la forme pharmaceutique et le mode d'administration souhaité, parmi les excipients habituels qui sont connus de l'homme du métier.

Dans les compositions pharmaceutiques de la présente invention pour l'administration orale, sublinguale, sous-cutanée, intramusculaire, intra-veineuse, topique, locale, intrathécale, intranasale, transdermique, pulmonaire, oculaire ou rectale, le principe actif de formule (I) ci-dessus, ou son solvat ou hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules, les chewing-gums et les solutions ou suspensions orales, les formes d'administration sublinguale, buccale, intratrachéale, intraoculaire, intranasale, par inhalation, les formes d'administration sous-cutanée, intramusculaire ou intraveineuse et les formes d'administration rectale ou vaginale. Pour l'application topique, on peut utiliser les composés selon l'invention dans des crèmes, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

Lesdites formes unitaires sont dosées pour permettre une administration journalière de 0,1 à 20 mg de principe actif par kg de poids corporel, selon la forme galénique.

Il peut y avoir des cas particuliers où des dosages plus élevés ou plus faibles sont appropriés, de tels dosages appartiennent également à l'invention. Selon la pratique habituelle, le dosage approprié à chaque patient est déterminé par le médecin selon le mode d'administration, le poids et la réponse dudit patient.

## Revendications

1. Composé répondant à la formule générale (I) : 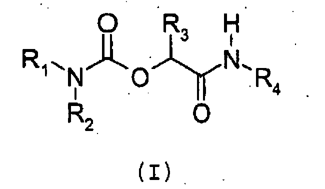 dans laquelle
R₁ représente un groupe C₅₋₂₆-alkyle, C₅₋₂₆-alkènyle, C₅₋₂₆-alkynyle ou un groupe carboné aliphatique polyinsaturé comprenant entre 5 et 26 atomes de carbone et comprenant au moins deux insaturations choisies parmi les doubles et les triples liaisons ;
R₂ représente un atome d'hydrogène ou un groupe C₁₋₆-alkyle ;
R₃ représente un atome d'hydrogène ou un groupe C₁₋₃-alkyle ;
R₄ représente un atome d'hydrogène ou un groupe C₁₋₄-alkyle, C₃₋₇-cycloalkyle, C₃₋₇-cycloalkyl-C₁₋₃₃-alkyle ;
les groupes alkyle, alkènyle, alkynyle et carboné aliphatique polyinsaturé pouvant être linéaires, ramifiés ou cycliques ;
à l'état de base, d'hydrate ou de solvat.

2. Composé de formule (I) selon la revendication 1, **caractérisé en ce que** :
- R₁ représente un groupe C₅₋₂₆-alkyle, un groupe C₅₋₂₆-alkènyle, ou un groupe carboné aliphatique polyinsaturé comprenant entre 5 et 26 atomes de carbone et comprenant au moins deux insaturations choisies parmi les doubles liaisons ;
- R₂ représente un atome d'hydrogène ou un groupe C₁₋₆-alkyle *;*
- R₃ représente un atome d'hydrogène ou un groupe C₁₋₃-alkyle ;
- R₄ représente un atome d'hydrogène ou un groupe C₁₋₄-alkyle, C₃₋₇-cycloalkyle, C₃₋₇-cycloalkyl-C₁₋₃-alkyle;
à l'état de base, d'hydrate ou de solvat.

3. Composé de formule (I) selon l'une quelconque des revendications 1 ou 2, **caractérisé en ce que** :
R₃ représente un atome d'hydrogène ou un groupe C₁₋₃-alkyle ;
R₄ représente un atome d'hydrogène ou un groupe C₁₋₄-alkyle ;
à l'état de base, d'hydrate ou de solvant.

4. Composé de formule (I) selon l'une quelconque des revendications 1 à 3, **caractérisé en ce que** :
- R₁ représente un groupe C₁₀₋₂₀-alkyle, C₁₀₋₂₀-alkènyle, C₁₀₋₂₀-alkynyle ou un groupe carboné aliphatique polyinsaturé comprenant entre 10 et 20 atomes de carbone et comprenant au moins deux insaturations choisies parmi les doubles et les triples liaisons ;
- R₂ représente un atome d'hydrogène ou un groupe C₁₋₆-alkyle ;
- R₃ représente un atome d'hydrogène ou un groupe C₁₋₃-alkyle ;
- R₄ représente un atome d'hydrogène ou un groupe C₁₋₄-alkyle, C₃₋₇-cycloalkyle, C₃₋₇-cycloalkyl-C₁₋₃-alkyle; à l'état de base, d'hydrate ou de solvat.

5. Composé de formule (I) selon l'une quelconque des revendications 1 à 4, **caractérisé en ce que** :
- R₁ représente un groupe C₁₀₋₂₀-alkyle, un groupe C₁₀₋₂₀-alkènyle ou un groupe carboné aliphatique polyinsaturé comprenant entre 10 et 20 atomes de carbone et comprenant au moins deux insaturations choisies parmi les doubles liaisons ;
- R₂ représente un atome d'hydrogène ou un groupe C₁₋₆-alkyle ;
- R₃ représente un atome d'hydrogène ou un groupe C₁₋₃-alkyle ;
- R₄ représente un atome hydrogène ou un groupe C₁₋₄-alkyle ;
à l'état de base, d'hydrate ou de solvant.

6. Composé de formule (I) selon l'une quelconque des revendications 1 à 5, **caractérisé en ce qu'**il s'agit de l'un des composés suivants :
- Pent-1-yl carbamate de 2-amino-1-méthyl-2-oxoéthyle
- (2E)-Hex-2-èn-1-yl carbamate de 2-(méthylamino)-2-oxoéthyle
- (2E)-Hex-2-èn-1-yl carbamate de 2-amino-2-oxoéthyle
- (2Z)-Hex-2-èn-1-yl carbamate de 2-amino-2-oxoéthyle
- (3Z)-Hex-3-èn-1-yl carbamate de 2-amino-2-oxoéthyle
- (3E)-Hex-3-èn-1-yl carbamate de 2-amino-2-oxoéthyle
- (3Z)-Hex-3-èn-1-yl carbamate de 2-(méthylamino)-2-oxoéthyle
- (3E)-Hex-3-èn-1-yl carbamate de 2-(méthylamino)-2-oxoéthyle
- (2Z)-Hex-2-èn-1-yl carbamate de 2-(méthylamino)-2-oxoéthyle
- Hex-1-yl carbamate de 2-(méthylamino)-2-oxoéthyle
- Hex-1-yl carbamate de 2-amino-1-méthyl-2-oxoéthyle
- Dihex-1-yl carbamate de 2-(méthylamino)-2-oxoéthyle
- Hept-1-yl carbamate de 2-(méthylamino)-2-oxoéthyle
- (2E)-oct-2-èn-1-yl carbamate de 2-(méthylamino)-2-oxoéthyle
- (2E)-Oct-2-èn-1-yl carbamate de 2-amino-2-oxoéthyle
- (3Z)-Oct-3-èn-1-yl carbamate de 2-(méthylamino)-2-oxoéthyle
- (3Z)-Oct-3-èn-1-yl carbamate de 2-amino-2-oxoéthyle
- (5Z)-Oct-5-èn-1-yl carbamate de 2-amino-2-oxoéthyle
- (5Z)-Oct-5-èn-1-yl carbamate de 2-(méthylamino)-2-oxoéthyle
- Oct-7-èn-1-yl carbamate de 2-amino-2-oxoéthyle
- Oct-7-èn-1-yl carbamate de 2-(méthylamino)-2-oxoéthyle
- Cyclooct-1-yl carbamate de 2-(méthylamino)-2-oxoéthyle
- Oct-1-yl carbamate de 2-amino-1-méthyl-2-oxoéthyle
- 1-Méthylhept-1-yl carbamate de 2-(méthylamino)-2-oxoéthyle
- Non-1-yl carbamate de 2-(méthylamino)-2-oxoéthyle
- Déc-1-yl carbamate de 2-(méthylamino)-2-oxoéthyle
- Déc-1-yl carbamate de 2-amino-1-méthyl-2-oxoéthyle
- 1-Méthyldéc-1-yl carbamate de 2-(méthylamino)-2-oxoéthyle
- Cyclododéc-1-yl carbamate de 2-(méthylamino)-2-dxoéthyle
- Dodéc-1-yl carbamate de 2-(méthylamino)-2-oxoéthyle
- (11Z)-Tetradéc-11-èn-1-yl carbamate de 2-amino-2-oxoéthyle
- (11Z)-Tetradéc-11-èn-1-yl carbamate de 2-(méthylamino)-2-oxoéthyle
- Tétradéc-1-yl carbamate de 2-(méthylamino)-2-oxoéthyle
- (9Z)-Hexadéc-9-èn-1-yl carbamate de 2-amino-2-oxoéthyle
- (9Z)-Hexadéc-9-èh-1-yl carbamate de 2-(méthylamino)-2-oxoéthyle
- 1-Méthyltridécyl carbamate de 2-(méthylamino)-2-oxoéthyle
- Pentadéc-1-yl carbamate de 2-(méthylamino)-2-oxoéthyle
- (6E,9E,12E)-Octadéca-6,9,12-trièn-1-yl carbamate de 2-(méthylamino)-2-oxoéthyle
- (6Z)-Octadéc-6-èn-1-yl carbamate de 2-amino-2-oxoéthyle
- (6Z)-Octadéc-6-èn-1-yl carbamate de 2-(méthylamino)-2-oxoéthyle
- (Méthyl)octadéc-1-yl carbamate de 2-(méthylamino)-2-oxoéthyle
- (4Z,7Z,10Z,13Z)-Nonadéca- 4,-7,10,13-tétraèn-1-yl carbamate de 2-amino-2-oxoéthyle
- Nonadéc-1-yl carbamate de 2-(méthylamino)-2-oxoéthyle
- (5Z,8Z,11Z,14Z)-Icosa-5,8,11,14-tétraèn-1-yl carbamate de 2-(méthylamino)-2-oxoéthyle
- (11E,14E,17E)-Icosa-11,14,17-trièn-1-yl carbamate de 2-(méthylamino)-2-oxoéthyle
- (11E,14E)-Icosa-11,14-dièn-1-yl carbamate de 2-(méthylamino)-2-oxoéthyle
- (11E)-Icosa-11-ên-1-yl carbamate de 2-(méthylamino)-2-oxoéthyle.

7. Composé de formule (I) selon l'une quelconque des revendications 1 à 5, **caractérisé en ce qu'**il s'agit de l'un des composés suivants :
- Déc-1-yl carbamate de 2-(méthylamino)-2-oxoéthyle
- 1-Méthyldéc-1-yl carbamate de 2-(méthylamino)-2-oxoéthyle
- Cyclododéc-1-yl carbamate de 2-(méthylamino)-2-oxoéthyle
- Dodéc-1-yl carbamate de 2-(méthylamino)-2-oxoéthyle
- (11Z)-Tetradéc-11-èn-1-yl carbamate de 2-amino-2-oxoéthyle
- (11Z)-Tetradéc-11-èn-1-yl carbamate de 2-(mêthylamino)-2-oxoéthyle
- Tétradéc-1-yl carbamate de 2-(méthylamino)-2-oxoéthyle
- (9Z)-Hexadéc-9-èn-1-yl carbamate de 2-amino-2-oxoéthyle
- (9Z)-Hexadéc-9-èn-1-yl carbamate de 2-(méthylamino)-2-oxoéthyle
- 1-Méthyltridécyl carbamate de 2-(méthylamino)-2-oxoéthyle
- Pentadéc-1-yl carbamate de 2-(méthylamino)-2-oxoéthyle
- (6E,9E,12E)-Octadéca-6,9,12-trièn-1-yl carbamate de 2-(méthylamino)-2-oxoéthyle
- (62)-Octàdéc-6-èn-1-yl carbamate de 2-(méthylamino)-2-oxoéthyle
- (5Z,BZ,11Z,14Z)-Icosa-5,8,11,14-tétraèn-1-yl carbamate de 2-(méthylamino)-2-oxoéthyle
- (11E,24E,17E)-Icosa-11,14,17-trièn-1-yl carbamate de 2-(méthylamino)-2-oxoéthyle
- (11E,14E)-Icosa-11,14-dièn-1-yl carbamate de 2-(méthylamino)-2-oxoéthyle.

8. Procédé de préparation d'un composé de formule (I) telle que définie dans l'une quelconque des revendications 1 à 7, comprenant l'étape consistant, à faire réagir une amine de formule générale R₁R₂NH (II), dans laquelle R₁ et R₂ sont tels que définis dans la formule générale (I) selon la revendication 1, avec un carbonate de formule générale (III) 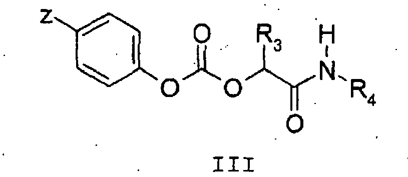 dans laquelle Z représente un atome d'hydrogène ou un groupe nitro et R₃ et R₄ sont tels que définis dans la formule générale (I) selon la revendication 1.

9. Procédé de préparation d'un composé de formule (I) telle que définie dans l'une quelconque des revendications 1 à 7 et dans laquelle R₂ représente un atome d'hydrogène, comprenant l'étape consistant à transformer le dérivé oxazoline-dione de formule générale (VI) 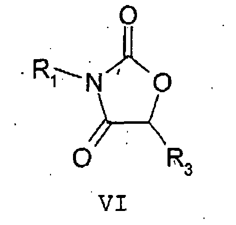 dans laquelle R₁ et R₃ sont tels que définis dans la formule générale (I) selon la revendication 1, par aminolyse au moyen d'une amine de formule générale R₄NH₂ dans laquelle R₄ est tel que défini dans la formule (I) selon la revendication 1.

10. Médicament, **caractérisé en ce qu'**il comprend un composé de formule (I) selon l'une quelconque des revendications 1 à 7, où un hydrate ou un solvat du composé de formule (I).

11. Composition pharmaceutique comprenant au moins un composé de formule (I) tel que défini dans l'une quelconque des revendications 1 à 7, à l'état de base, d'hydrate ou de solvat pharmaceutiquement acceptable et éventuellement un ou plusieurs excipients pharmaceutiquement acceptables.

12. Composé de formule (I) tel que défini dans l'une quelconque des revendications 1 à 7, à l'état de base, d'hydrate ou de solvat pharmaceutiquement acceptable, pour son utilisation comme médicament.

13. Utilisation d'un composé de formule (I) selon l'une quelconque des revendications 1 à 7, à l'état de base, d'hydrate ou de solvat pharmaceutiquement acceptable, pour la préparation d'un médicament destiné à prévenir ou à traiter les douleurs aiguës ou chroniques, les vertiges, les vomissements, les nausées, les troubles du comportement alimentaire, les pathologies neurologiques et psychiatriques, les maladies neuro-d'égénératives aiguës ou chroniques, l'épilepsie, les troubles du sommeil, les maladies cardiovasculaires, l'ischémie rénale, les cancers, les désordres du système immunitaire, les maladies allergiques, les maladies infectieuses parasitaires, virales ou bactériennes, les maladies inflammatoires, l'ostéoporose, les affections oculaires, les affections pulmonaires, les maladies gastro-intestinales ou l'incontinence urinaire.

## Claims

1. Compound corresponding to the general formula (I): 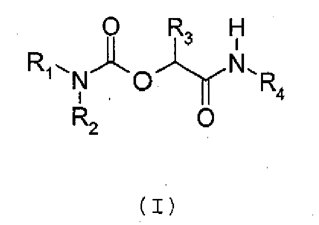 in which
R¹ represents a C₅₋₂₆-alkyl, C₅₋₂₆-alkenyl or C₅₋₂₆-alkynyl group or a polyunsaturated aliphatic carbon group comprising between 5 and 26 carbon atoms and comprising at least two unsaturations chosen from double and triple bonds;
R₂ represents a hydrogen atom or a C₁₋₆-alkyl group;
R₃ represents a hydrogen atom or a C₁₋₃-alkyl group;
R₄ represents a hydrogen atom or a C₁₋₄-alkyl, C₃₋₇-cycloalkyl or C₃₋₇-cycloalkyl-C₁₋₃-alkyl groups;
it being possible for the alkyl, alkenyl, alkynyl and polyunsaturated aliphatic carbon groups to be linear, branched or cyclic;
in the base, hydrate or solvate form.

2. Compound of formula (I) according to Claim 1, **characterized in that**:
- R₁ represents a C₅₋₂₆-alkyl group, a C₅₋₂₆-alkenyl group or a polyunsaturated aliphatic carbon group comprising between 5 and 26 carbon atoms and comprising at least two unsaturations chosen from double bonds;
- R₂ represents a hydrogen atom or a C₁₋₆-alkyl group;
- R₃ represents a hydrogen atom or a C₁₋₃-alkyl group;
- R₄ represents a hydrogen atom or a C₁₋₄-alkyl, C₃₋₇-cycloalkyl or C₃₋₇-cycloalkyl-C₁₋₃-alkyl group ;
in the base, hydrate or solvate form.

3. Compound of formula (I) according to either one of Claims 1 and 2, **characterized in that**:
R₃ represents a hydrogen atom or a C₁₋₃-alkyle group;
R₄ represents a hydrogen atom or a C₁₋₄-alkyle group;
in the base, hydrate or solvate form.

4. Compound of formula (I) according to any one of Claims 1 to 3, **characterized in that**:
- R₂ represents, a C₁₀₋₂₀-alkyl, C₁₀₋₂₀-alkenyl or C₁₀₋₂₀-alkynyl group or a polyunsaturated aliphatic carbon group comprising between 10 and 20 carbon atoms and comprising at least two unsaturations chosen from double and triple bonds;
- R₂ represents a hydrogen atom or a C₁₋₆-alkyl group;
- R₃ represents a hydrogen atom or a C₁₋₃-alkyl group;
- R₄ represents a hydrogen atom or a C₁₋₄-alkyl, C₃₋₇-cycloalkyl or C₃₋₇-cycloalkyl-C₁₋₃-alkyl group;
in the base, hydrate or solvate form.

5. Compound of formula (I) according to any one of Claims 1 to 4, **characterized in that**:
- R₁ represents a C₁₀₋₂₀-alkyl group, a C₁₀₋₂₀-alkenyl group or a polyunsaturated aliphatic carbon group comprising between 10 and 20 carbon atoms and comprising at least two unsaturations chosen from double bonds;
- R₂ represents a hydrogen atom or a C₁₋₆-alkyl group;
- R₃ represents a hydrogen atom or a C₁₋₃-alkyl group;
- R₄ represents a hydrogen atom or a C₁₋₄-alkyl group;
in the base, hydrate or solvate form.

6. Compound of formula (I) according to any one of Claims 1 to 5, **characterized in that** it is one of the following compounds:
- 2-Amino-1-methyl-2-oxoethyl (pent-1-yl)carbamate
- 2-(Methylamino)-2-oxoethyl ((2E)-hex-2-en-1-yl)carbamate
- 2-Amino-2-oxoethyl ((2E)-hex-2-en-1-yl)carbamate
- 2-Amino-2-oxoethyl ((2Z)-hex-2-en-1-yl)carbamate
- 2-Amino-2-oxoethyl ((3Z)-hex-3-en-1-yl)carbamate
- 2-Amino-2-oxoethyl ((3E)-hex-3-en-1-yl)carbamate
- 2-(Methylamino)-2-oxoethyl ((3Z)-hex-3-en-1-yl)carbamate
- 2-(Methylamino)-2-oxoethyl ((3E)-hex-3-en-1-yl)carbamate
- 2-(Methylamino)-2-oxoethyl ((2Z)-hex-2-en-1-yl)carbamate
- 2-(Methylamino)-2-oxoethyl (hex-1-yl)carbamate 2-Amino-1-methyl-2-oxoethyl(hex-1-yl)carbamate
- 2-(Methylamino)-2-oxoethyl(dihex-7-yl)carbamate
- 2-(Methylamino)-2-oxoethyl (hept-1-yl)carbamate
- 2-(Methylamino)-2-oxoethyl ((2E)-oct-2-en-1-yl)carbamate
- 2-Amino-2-oxoethyl ((2E)-oct-2-en-1-yl)carbamate
- 2-(Methylamino)-2-oxoethyl ((3Z)-oct-3-en-1-yl)carbamate
- 2-Amino-2-oxoethyl ((3Z)-oct-3-en-1-yl) carbamate
- 2-Amino-2-oxoethyl ((5Z)-oct-5-en-1-yl)carbamate
- 2-(Methylamino)-2-oxoethyl ((5Z)-oct-5-en-1-yl) carbamate
- 2-Amino-2-oxoethyl (oct-7-en-1-yl)carbamate
- 2-(Methylamino)-2-oxoethyl (oct-7-en-1-yl)carbamate
- 2-(Methylamino)-2-oxoethyl (cyclooct-1-yl)carbamate
- 2-Amino-1-methyl-2-oxoethyl (oct-1-yl)carbamate
- 2-(Methylamino)-2-oxoethyl (1-methylhept-1-yl)carbamate
- 2-(Methylamino)-2-oxoethyl (non-1-yl)carbamate
- 2-(Methylamino)-2-oxoethyl (dec-1-yl)carbamate
- 2-Amino-1-methyl-2-oxoethyl (dec-1-yl)carbamate
- 2-(Methylamino)-2-oxoethyl (1-methyldec-1-yl)carbamate
- 2-(Methylamino)-2-oxoethyl (cyclododec-1-yl)carbamate
- 2-(Methylamino)-2-oxoethyl (dodec-1-yl)carbamate
- 2-Amino-2-oxoethyl ((11Z)-tetradec-11-en-1-yl)carbamate
- 2-(Methylamino)-2-oxoethyl ((11Z)-tetradec-11-en-7-yl)carbamate
- 2-(Methylamino)-2-oxoethyl (tetradec-1-yl)carbamate
- 2-Amino-2-oxoethyl ((9Z)-hexadec-9-en-1-yl)carbamate
- 2-(Methylamino)-2-oxoethyl ((9Z)-hexadec-9-en-1-yl)carbamate
- 2-(Methylamino)-2-oxoethy(1-methyltridecyl)carbamate 2-(Methylamino)-2-oxoethyl (pentadec-1-yl)carbamate
- 2-(Methylamino)-2-oxoethyl ((6E,9E,12E)-octadeca-6,9,12-trien-1-yl)carbamate
- 2-Amino-2-oxoethyl ((6Z)-octadec-6-en-1-yl)carbamate
- 2-(Methylamino)-2-oxoethyl ((6Z)-octadec-6-en-1-yl)carbamate
- 2-(Methylamino)-2-oxoethyl ((methyl)octadec-1-yl)carbamate
- 2-Amino-2-oxoethyl ((4Z,7Z,10Z,13Z)-nonadeca-4,7,1a,13-tetraen-1-yl)carbamate
- 2-(Methylamino)-2-oxoethyl (nonadec-1-yl)carbamate
- 2-(Methylamino)-2-oxoethyl ((5Z,8Z,11Z,14Z)-icosa-5,8,11,14-tetraen-1-yl)carbamate
- 2-(Methylamino)-2-oxoethyl ((11E,14E,17E)-icosa-11,14,17-trien-1-yl)carbamate
- 2-(Methylamino)-2-oxoethyl ((11E, 14E)-icosa-11,14-dien-1-yl)carbamate
- 2-(Methylamino)-2-oxoethyl ((11E)-icosa-11-en-1-yl)carbamate.

7. Compound of formula (I) according to any one of Claims 1 to 5, **characterized in that** it is one of the following compounds:
- 2-(Methylamino)-2-oxoethyl (dec-1-yl)carbamate
- 2-(Methylamino)-2-oxoethyl (1-methyldec-1-yl)carbamate
- 2-(Methylamino)-2-oxoethyl (cyclododec-1-yl)carbamate
- 2-(Methylamino)-2-oxoethyl (dodec-1-yl)carbamate
- 2-Amino-2-oxoethyl ((11Z)-tetradec-11-en-1-yl)carbamate
- 2-(Methylamino)-2-oxoethyl ((11Z)-tetradec-11-en-1-yl)carbamate
- 2-(Methylamino)-2-oxoethyl (tetradec-1-yl)carbamate
- 2-Amino-2-oxoethyl ((9Z)-hexadec-9-en-1-yl)carbamate
- 2-(Methylamino)-2-oxoethyl ((9Z)-hexadec-9-en-1-yl)carbamate
- 2-(Methylamino)-2-oxoethy(1-methyltridecyl)carbamate
- 2-(Methylamino)-2-oxoethyl (pentadec-1-yl)carbamate
- 2-(Methylamino)-2-oxoethyl ((6E,9E,12E)-actadeca-6,9,12-trien-1-yl)carbamate
- 2-(Methylamino)-2-oxoethyl ((6Z)-actadec-6-en-1-yl)carbamate
- 2-(Methylamino)-2-oxoethyl ((5Z,8Z,11Z,14Z)-icosa-5,8,11,14-tetraen-1-yl)carbamate
- 2-(Methylamino)-2-oxoethyl ((11E,14E,17E)-icosa-11,14,17-trien-1-yl)carbamate
- 2-(Methylamino)-2-oxoethyl ((11E,14E)-icosa-11,14-dien-1-yl)carbamate.

8. Process for the preparation of a compound of formula (I) as defined in any one of Claims 1 to 7, comprising the stage consisting in reacting an amine of general formula R₁R₂NH (II), in which R₁ and R₂ are as defined in the' general formula (I) according to Claim 1, with a carbonate of general formula (III) 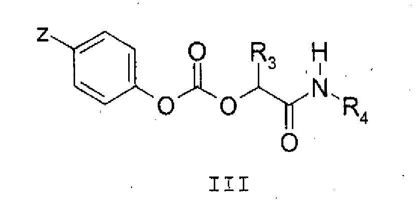 in which Z represents a hydrogen atom or a nitro group and R₃ and R₄ are as defined in the general formula (I) according to Claim 1.

9. Process for the preparation of a compound of formula (I) as defined in any one of Claims 1 to 7 and in which R₂ represents a hydrogen atom, comprising the stage consisting in converting the oxazolidinedione derivative of general formula (VI) 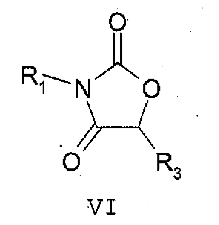 in which R₁ and R₃ are as defined in the general formula (I) according to Claim 1, by aminolysis using an amine of general formula R₄NH₂ in which R₄ is as defined in the formula (I) according to Claim 1.

10. Medicament, **characterized in that** it comprises a compound of formula (I) according to any one of Claims 1 to 7 or a hydrate or a solvate of the compound of formula (I).

11. Pharmaceutical composition comprising at least one compound of formula (I) as defined in any one of Claims 1 to 7, in the pharmaceutically acceptable base, hydrate or solvate form, and optionally one or more pharmaceutically acceptable excipients.

12. Compound of formula (I) as defined in any one of Claims 1 to 7, in the pharmaceutically acceptable base, hydrate or solvate form, for its use as medicament.

13. Use of a compound of formula (I) according to any one of Claims 1 to 7, in the pharmaceutically acceptable base, hydrate or solvate form, in the preparation of a medicament intended to prevent or treat acute or chronic pain, dizziness, vomiting, nausea, eating disorders, neurological and psychiatric pathologies, acute or chronic neurodegenerative diseases, epilepsy, sleep disorders, cardiovascular diseases, renal ischaemia, cancers, disorders of the immune system, allergic diseases, parasitic, viral or bacterial infectious diseases, inflammatory diseases, osteoporosis, eye conditions, pulmonary conditions, gastrointestinal diseases or urinary incontinence.

## Patentansprüche

1. Verbindung der allgemeinen Formel (I): 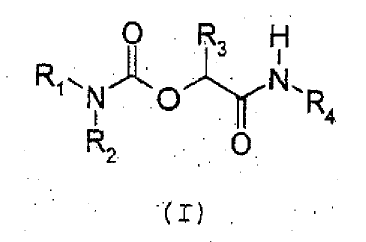 worin
R₁ für eine C₅₋₂₆-Alkyl-, C₅₋₂₆-Alkenyl oder C₅₋₂₆-Alkinylgruppe oder eine mehrfach ungesättigte aliphatische Kohlenstoffgruppe mit zwischen 5 und 26 Kohlenstoffatomen und mindestens zwei unter Doppel- und Dreifachbindungen ausgewählten Ungesättigtheiten steht;
R₂ für ein Wasserstoffatom oder eine C₁₋₆-Alkylgruppe steht;
R₃ für ein Wasserstoffatom oder eine C₁₋₃-Alkylgruppe steht;
R₄ für ein Wasserstoffatom oder eine C₁₋₄-Alkyl-, C₃₋₇-Cycloalkyl- oder C₃₋₇-Cycloalkyl-C₁₋₃-alkylgruppe steht;
wobei die Alkyl-, Alkenyl-, Alkinyl- und mehrfach ungesättigten aliphatischen Kohlenstoffgruppen linear, verzweigt oder cyclisch sein können;
in Basen-, Hydrat- oder Solvatform.

2. Verbindung der Formel (I) nach Anspruch 1, **dadurch gekennzeichnet, daß**:
- R₁ für eine C₅₋₂₆-Alkylgruppe, eine C₅₋₂₆-Alkenyl-gruppe oder eine mehrfach ungesättigte aliphatische Kohlenstoffgruppe mit zwischen 5 und 26 Kohlenstoffatomen und mindestens zwei unter Doppelbindungen ausgewählten Ungesättigtheiten steht;
- R₂ für ein Wasserstoffatom oder eine C₁₋₆-Alkylgruppe steht;
- R₃ für ein Wasserstoffatom oder eine C₁₋₃-Alkylgruppe steht;
- R₄ für ein Wasserstoffatom oder eine C₁₋₄-Alkyl-, C₃₋₇-Cycloalkyl- oder C₃₋₇-Cycloalkyl-C₁₋₃-alkylgruppe steht;
in Basen-, Hydrat- oder Solvatform.

3. Verbindung der Formel (I) nach einem der Ansprüche 1 und 2, **dadurch gekennzeichnet, daß**:
R₃ für ein Wasserstoffatom oder eine C₁₋₃-Alkyl-kruppe steht;
R₄ für ein Wasserstoffatom oder eine C₁₋₄-Alkylgruppe steht;
in Basen-, Hydrat- oder Solvatform.

4. Verbindung der Formel (I) nach einem der Ansprüche 1 bis 3, **dadurch gekennzeichnet, daß**:
- R₁ für eine C₁₀₋₂₀-Alkyl-, C₁₀₋₂₀-Alkenyl- oder C₁₀₋₂₀-Alkinylgruppe oder eine mehrfach ungesättigte aliphatische Kohlenstoffgruppe mit zwischen 10 und 20 Kohlenstöffatomen und mindestens zwei unter Doppel- und Dreifachbindungen ausgewählten Ungesättigtheiten steht;
- R₂ für ein Wasserstoffatom oder eine C₁₋₆-Alkylgruppe steht;
- R₃ für ein Wasserstoffatom oder eine C₁₋₃-Alkylgruppe steht;
- R₄ für ein Wasserstoffatom oder eine C₁₋₄-Alkyl-, C₃₋₇-Cycloalkyl- oder C₃₋₇-Cycloalkyl-C₁₋₃-alkylgruppe steht;
in Basen-, Hydrat- oder Solvatform.

5. Verbindung der Formel (I) nach einem der Ansprüche 1 bis 4, **dadurch gekennzeichnet, daß**:
- R₁ für eine C₁₀₋₂₀-Alkylgruppe, eine C₁₀₋₂₀-Alkenylgruppe oder eine mehrfach ungesättigte aliphatische Kohlenstoffgruppe mit zwischen 10 und 20 Kohlenstoffatomen und mindestens zwei unter Doppelbindungen ausgewählten Ungesättigtheiten steht;
- R₂ für ein Wasserstoffatom oder eine C₁₋₆-Alkylgruppe steht;
- R₃ für ein Wasserstoffatom oder eine C₁₋₃-Alkylgruppe steht;
- R₄ für ein Wasserstoffatom oder eine C₁₋₄-Alkylgruppe steht;
in Basen-, Hydrat- oder Solvatform.

6. Verbindung der Formel (I) nach einem der Ansprüche 1 bis 5, **dadurch gekennzeichnet, daß** es sich um eine der folgenden Verbindungen handelt:
- Pent-1-ylcarbamidsäure-2-amino-1-methyl-2-oxoethylester
- (2E)-Hex-2-en-1-ylcarbamidsäure-2-(methylamino)-2-oxoethylester
- (2E)-Hex-2-en-1-ylcarbamidsäure-2-amino-2-oxoethylester
- (2Z)-Hex-2-en-1-ylcarbamidsäure-2-amino-2-oxoethylester
- (3Z)-Hex-3-en-1-ylcarbamidsäure-2-amino-2-oxoethylester
- (3E)-Hex-3-en-1-ylcarbamidsäure-2-amino-2-oxoethylester
- (3Z)-Hex-3-en-1-ylcarbamidsäure-2-(methylamino)-2-oxoethylester
- (3E)-Hex-3-en-1-ylcarbamidsäure-2-(methylamino)-2-, oxoethylester
- (2Z)-Hex-2-en-1-ylcarbamidsäure-2-(methylamino)-2-oxoethylester
- Hex-1-ylcarbamidsäure-2-(methylamino)-2-oxoethylester
- Hex-1-ylcarbamidsäure-2-amino-1-methyl-2-oxoethylester
- Dihex-1-ylcarbamidsäure-2-(methylamino)-2-oxo-ethylester
- Hept-1-ylcarbamidsäure-2-(methylamino)-2-oxoethylester
- (2E)-Oct-2-en-2-ylcarbamidsäure-2-(methylamino)-2-oxoethylester
- (2E)-Oct-2-en-1-ylcarbamidsäure-2-amino-2-oxoethylester
- (3Z)-Oct-3-en-ylcarbamidsäure-2-(methylamino)-2-oxoethylester
- (3Z)-Oct-3-en-1-ylcarbamidsäure-2-amino-2-oxoethylester
- (5Z)-Oct-5-en-1-ylcarbamidsäüre-2-amino-2-oxoethylester
- (5Z)-Oct-5-en-1-ylcarbamidsäure-2-(methylamino)-2-oxoethylester
- Oct-7-en-1-ylcarbamidsäure-2-amino-2-oxoethylester
- Oct-7-en-1-ylcarbamidsäüre-2-(methylamino)-2-oxoethylester
- Cyclooct-1-ylcarbamidsäure-2-(methylamino)-2-oxoethylester
- Oct-1-ylcarbamidsäure-2-amino-1-methyl-2-oxoethylester
- 1-Methylhept-1-ylcarbamidsäure-2-(methylamino)-2-oxoethylester
- Non-1-ylcarbamidsäure-2-(methylamino)-2-oxoethylester
- Dec-1-ylcarbamidsäure-2-(methylamino)-2-oxoethylester
- Dec-1-ylcarbamidsäure-2-amino-1-methyl-2-oxoethylester
- 1-Methyldec-1-ylcarbamidsäure-2-(methylamino)-2-oxoethylester
- Cyclododec-1-ylcarbamidsäure-2-(methylamino)-2-oxoethylester
- Dodec-2-ylcarbamidsäure-2-(methylamino)-2-oxoethylester
- (11Z)-Tetradec-11-en-1-ylcarbamidsäure-2-amino-2-oxoethylester
- (11Z)-Tetradec-11-en-1-ylcarbamidsäure-2-(methylamino)-2-oxoethylester
- Tetradec-1-ylcarbamidsäure-2-(methylamino)-2-oxoethylester
- (9Z)-Hexadec-9-en-1-ylcarbamidsäure-2-amino-2-oxoethylester
- (9Z)-Hexadec-9-en-1-ylcarbamidsäure-2-(methylamino)-2-oxoethylester
- 1-Methyltridecylcarbamidsäure-2-(methylamino)-2-oxoethylester
- Pentadec-1-ylcarbamidsäure-2-(methylamino)-2-oxoethylester
- (6E,9E,12E)-Octadeca-6,9,12-trien-1-ylcarbamid-säure-2-(methylamino)-2-oxoethylester
- (6Z)-Octadec-6-en-1-ylcarbamidsäure-2-amino-2-oxoethylester
- (6Z)-Octadec-6-en-1-ylcarbamidsäure-2-(methylamino)-2-oxoethylester
- (Methyl)octadec-1-ylcarbamidsäure-2-(methylamino)-2-oxoethylester
- (4Z,7Z,10Z,13Z)-Nonadeca-4,7,10,13-tetraen-1-ylcarbamidsäure-2-amino-2-oxoethylester
- Nonadec-1-ylcarbamidsäure-2-(methylamino)-2-oxo-ethylester
- (5Z,8Z,11Z,14Z)-Icosa-5,8,11,14-tetraen-1-ylcarbamidsäure-2-(methylamino)-2-oxoethylester
- (11E,14E,17E)-Icosa-11,14,17-trien-1-ylcarbamid-säure-2-(methylamino)-2-oxoethylester
- (11E,14E)-Icosa-11,14-dien-1-ylcarbamidsäure-2-(methylamino)-2-oxoethylester
- (11E)-Icasa-11-en-1-ylcarbamidsäure-2-(methylamino)-2-oxoethyl.

7. Verbindung der Formel (I) nach einem der Ansprüche 1 bis 5, **dadurch gekennzeichnet, daß** es sich um eine der folgenden Verbindungen handelt:
- Dec-1-ylcarbamidsäure-2-(methylamino)-2-oxoethylester
- 1-Methyldec-1-ylcarbamidsäure-2-(methylamino)-2-oxoethylester
- Cyclododec-1-ylcarbamidsäure-2-(methylamino)-2-oxoethylester
- Dodec-1-ylcarbamidsäure-2-(methylamino)-2-oxoethylester
- (11Z)-Tetradec-11-en-1-ylcarbamidsäure-2-amino-2-oxoethylester
- (11Z)-Tetradec-11-en-1-ylcarbamidsäure-2-(methylamino)-2-oxoethylester
- Tetradec-1-ylcarbamidsäure-2-(methylamino)-2-oxoethylester
- (9Z)-Hexadec-9-en-1-ylcarbamidsäure-2-amino-2-oxoethylester
- (9Z)-Hexadec-9-en-1-ylcarbamidsäure-2-(methylamino)-2-oxoethylester
- 1-Methyltridecylcarbamidsäure-2-(methylamino)-2-oxoethylester
- Pentadec-1-ylcarbamidsäure-2-(methylamino)-2-oxoethylester
- (6E,9E,12E)-Octadeca-6,9,12-trien-1-ylcarbamid-säure-2-(methylamino)-2-oxoethylester
- (6Z)-Octadec-6-en-1-ylcarbamidsäure-2-(methylamino)-2-oxoethylester
- (5Z,8Z,11Z,14Z)-Icosa-5,8,11,14-tetraen-1-ylcarbamidsäure-2-(methylamino)-2-oxoethylester
- (11E,14E,17E)-Icosa-11,14,17-trien-1-ylcarbamidsäure-2-(methylamino)-2-oxoethylester
- (11E,14E)-Icosa-11,14-dien-1-ylcarbamidsäure-2-(methylamino)-2-oxoethylester.

8. Verfahren zur Herstellung einer Verbindung der Formel (I) nach einem der Ansprüche 1 bis 7, bei dem man ein Amin der allgemeinen Formel R₁R₂NH (II), worin R₁ und R₂ die unter der allgemeinen Formel (I) gemäß Anspruch 1 angegebene Bedeutung besitzen, mit einem Kohlensäureester der allgemeinen Formel (III) 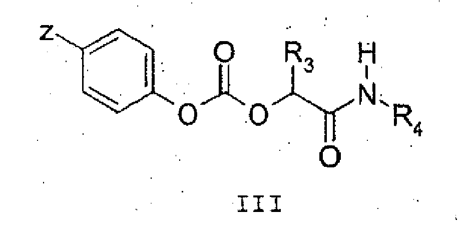 worin Z für ein Wasserstoffatom oder eine Nitrogruppe steht und R₃ und R₄ die unter der allgemeinen Formel (I) gemäß Anspruch 1 angegebene Bedeutung besitzen, umsetzt.

9. Verfahren zur Verstellung einer Verbindung der Formel (I) nach einem der Ansprüche 1 bis 7, worin R₂ für ein Wasserstoffatom steht, bei dem man das Oxazolindionderivat der allgemeinen Formel (VI) 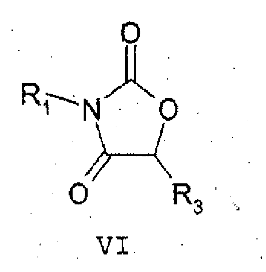 worin R₁ und R₃ die unter der allgemeinen Formel (I) gemäß Anspruch 1 angegebene Bedeutung besitzen, durch Aminolyse mit einem Amin der allgemeinen Formel R₄NH₂, worin R₄ die unter der allgemeinen Formel (I) gemäß Anspruch 1 angegebene Bedeutung besitzt, umwandelt.

10. Arzneimittel, **dadurch gekennzeichnet, daß** es eine Verbindung der Formel (I) nach einem der Ansprüche 1 bis 7 oder ein Hydrat oder ein Solvat der Verbindung der Formel (I) umfaßt.

11. Pharmazeutische Zusammensetzung, die mindestens eine Verbindung der Formel (I) nach einem der Ansprüche 1 bis 7 in pharmazeutisch unbedenklicher Basen-, Hydrat- oder Solvatform und gegebenenfalls einen oder mehrere pharmazeutisch unbedenkliche Hilfsstoffe umfaßt.

12. Verbindung der Formel (I) nach einem der Ansprüche 1 bis 7 in pharmazeutisch unbedenklicher Basen-, Hydrat- oder Solvatform zur Verwendung als Arzneimittel.

13. Verwendung einer Verbindung der Formel (I) nach einem der Ansprüche 1 bis 7 in pharmazeutisch unbedenklicher Basen-, Hydrat- oder Solvatform zur Herstellung eines Arzneimittels zur Prävention oder Behandlung von akuten oder chronischen Schmerzen, Schwindel, Erbrechen, Übelkeit, Eßstörungen, neurologischen und psychiatrischen Pathologien, akuten und chronischen neurodegenerativen Erkrankungen, Epilepsie, Schlafstörungen, Herz-Kreislauf-Erkrankungen, Nierenischämie, Krebs, Störungen des Immunsystems, allergischen Erkrankungen, parasitären, viralen oder bakteriellen Infektionserkrankungen, entzündlichen Erkrankungen, Osteoporose, Augenleiden, Lungenleiden, Magen-Darm-Erkrankungen oder Harninkontinenz.
